# EUROPÄISCHE PATENTSCHRIFT

(11) **EP 1 808 128 B1**
(45) Veröffentlichungstag und Bekanntmachung des Hinweises auf die Patenterteilung: **02.07.2008**
(21) Anmeldenummer: 06100216.8
(22) Anmeldetag: 10.01.2006
(51) Int. Cl.: A61B 5/15

(54) **Stechhilfe mit Wiederverwendungsschutz**
Puncturing auxiliary device with protection against re-use
Dispositif auxiliaire de piqûre avec protection contre réutilisation

(43) Veröffentlichungstag der Anmeldung: 18.07.2007
(60) Teilanmeldung: 07122066.9
(73) Patentinhaber: F. Hoffmann-la Roche AG, 4070 Basle (CH); Roche Diagnostics GmbH, 68305 Mannheim (DE)
(72) Erfinder: Schosnig, Stefan, 69493 Hirschberg-Großsachsen (DE); Handel, Wolfgang, 68305 Mannheim (DE); Jost, Wolfgang, 67482 Boebingen (DE); Kolonko, Lydia, 64646 Heppenheim (DE); List, Hans, 64754 Hesseneck-Kailbach (DE); Griffis, Jack, Decatur, Georgia 30033 (US); Leutz, Brian, Suwannee, Georgia 30024 (US); Stout, Jeff, Smyrna, Georgia 30080 (US); Kennedy, Gwen, Ellenwood GA 30294 (US); Koeppel, Bradley, Smyrna GA 30082 (US); Noble, Jeff, McDonough, Georgia 30253 (US)
(74) Vertreter: Huhn, Michael

(56) Entgegenhaltungen:
- EP-A- 1 371 329

## Beschreibung

Die vorliegende Erfindung betrifft eine Einmal-Stechhilfe, deren Wiederverwendung durch ein Blockierelement unterbunden wird.

Eine Entnahme von Körperflüssigkeiten, insbesondere von Blut, wird vor allem mit dem Ziel einer nachfolgenden Analyse durchgeführt, um eine Diagnose von Krankheiten oder die Überwachung des Stoffwechselzustandes eines Patienten zu ermöglichen. Insbesondere von Diabetikern wird eine solche Entnahme durchgeführt, um die Blutzuckerkonzentration zu bestimmen. Um für Diagnosezwecke eine Entnahme von nur geringen Mengen Blut vorzunehmen, werden üblicherweise sterile, scharfe Lanzetten verwendet, die z. B. von Krankenhauspersonal oder dem Patienten selbst bei dem Patienten in die Fingerbeere oder auch andere Körperteile kurz eingestochen werden. Vor allem im Bereich des so genannten "Home-Monitoring", wobei medizinische Laien selbst einfache Analysen des Blutes durchführen, werden Lanzetten und dazu passende Geräte (so genannte Blutentnahmegeräte, Blutlanzettenvorrichtungen oder - wie sie im Folgenden genannt werden sollen - Stechhilfen), angeboten, die eine möglichst schmerzarme und reproduzierbare Blutgewinnung ermöglichen.

Einmal-Stechhilfen, die einen Mechanismus zum Verhindern einer Mehrfachverwendung enthalten, sind im Stand der Technik bekannt. Eine Mehrfachverwendung einer Stechhilfe birgt die Gefahr einer Kontamination oder Infektion des Benutzers durch an der Lanzette der Stechhilfe vorhandene Blutreste.

US 6,764,496 B2 und US 6,514,270 B1 beziehen sich jeweils auf eine Lanzettenvorrichtung für den Einmalgebrauch, mit einer Lanzette im Inneren eines Gehäuses, die sich zwischen einer gespannten Position und einer Einstechposition bewegen kann. Ein Begrenzungsaufbau ist so ausgebildet, dass er in die Lanzette eingreift und dadurch verhindert, dass die Lanzette nach einer Bewegung in die Einstechposition wieder zurück in die gespannte Position bewegt wird.

US 6,719,771 B1 betrifft eine Blutentnahmeeinrichtung, umfassend eine Hülse mit einer federbelasteten Lanzette, die durch einen Auslöser von einer gespannten rückwärtigen Position freisetzbar ist, um augenblicklich ihre Spitze aus dem vorwärtigen Ende der Hülse hinausragen zu lassen. Die Lanzette weist ein ausbiegbares oder lösbares Anhängsel auf, welches mit der Hülse zum Eingriff führbar ist und die Lanzette gegen den Druck der Feder hält, wobei das Anhängsel sich rückwärtig von der Lanzette erstreckt und einen Vorsprung hintergreift, welcher an einem inneren Aufbau der Hülse vorgesehen ist. Die Freisetzung der Lanzette erfolgt durch den Auslöser, welcher integral mit der Hülse ausgebildet ist und quer zur Hülse gedrückt wird, um das Anhängsel zu entriegeln. Ferner kann der Auslöser, wenn er betätigt wird, einen Schnappeingriff mit der Hülse besitzen, um diese in ihrer betätigten Position zurückzuhalten.

EP 1 371 329 A1 hat eine Stechhilfe zum Gegenstand, die ein Gehäuse mit einer Öffnung umfasst, in dem ein Lanzettenhalter mit einer Lanzette beweglich gelagert ist. Der Lanzettenhalter ist mit einem Federelement verbunden und beinhaltet mindestens ein Lagerelement. In dem Gehäuse ist mindestens eine Auflagefläche in der Weise angeordnet, dass das Lagerelement in einer ersten Position des Lanzettenhalters auf der Auflagefläche aufliegt. Die Stechhilfe umfasst ferner eine Auslöseeinheit, durch die der Lanzettenhalter in eine zweite Position überführt wird, wobei eine Auslösetaste der Auslöseeinheit eine lineare Bewegung ausführt und die lineare Bewegung in eine relative Drehbewegung des Lagerelements und der Auflagefläche zueinander überführt wird. In der zweiten Position des Lanzettenhalters fällt das Lagerelement von der Auflagefläche, wobei sich das vor der Fallbewegung gespannte Federelement zumindest teilweise entspannt und den Lanzettenhalter relativ zum Gehäuse bewegt, so dass die Spitze der Lanzette aus der Öffnung des Gehäuses austritt.

Diese Stechhilfe gemäß EP 1 371 329 A1 hat den Nachteil, dass unter Verwendung eines geeigneten Werkzeuges in Form eines länglichen Elements, das durch die Öffnung in das Gehäuse eingeführt werden kann, das Lagerelement durch eine lineare und anschließende rotierende Bewegung wieder auf der Auflagefläche positioniert werden kann. Je nach Gestaltung des Sterilschutzes kann sogar dieser als Werkzeug zum erneuten Spannen der Stechhilfe verwendet werden. Somit ist eine unerwünschte Wiederverwendung der Stechhilfe möglich. Für diese Stechhilfe wird ein Wiederverwendungsschutz vorgeschlagen, bei der das Lagerelement von einem Federelement nach dem Stechvorgang von unten gegen die Auflagefläche gedrückt wird. Durch die Verwendung eines Werkzeuges kann jedoch auch bei einem solchen Wiederverwendungsschutz ein erneutes Spannen der Stechhilfe nicht verhindert werden.

Die Aufgabe der vorliegenden Erfindung ist es daher, bei einer Stechhilfe gemäß EP 1 371 329 A1 eine Wiederverwendung wirksam zu verhindern.

Diese Aufgabe wird erfindungsgemäß gelöst durch eine Stechhilfe umfassend
- ein Gehäuse mit einer Öffnung, in dem ein Lanzettenhalter mit einer Lanzette beweglich gelagert ist, wobei der Lanzettenhalter mit einem Federelement verbunden ist und der Lanzettenhalter mindestens ein Lagerelement umfasst, und in dem Gehäuse mindestens eine Auflagefläche in der Weise angeordnet ist, dass das Lagerelement in einer ersten Position des Lanzettenhalters auf der Auflagefläche aufliegt, sowie
- eine Auslöseeinheit, durch die der Lanzettenhalter durch Betätigen einer Auslösetaste mittels einer relativen Drehbewegung des Lagerelements und der Auflagefläche zueinander in eine zweite Position überführt wird, so dass das Lagerelement in der zweiten Position des Lanzettenhalters von der Auflagefläche fällt, wobei sich das vor der Fallbewegung gespannte Federelement zumindest teilweise entspannt und den Lanzettenhalter relativ zum Gehäuse bewegt, so dass die Spitze der Lanzette aus der Öffnung des Gehäuses austritt,
wobei die Stechhilfe ein Blockierelement umfasst, das so angeordnet ist, dass es eine relative Drehbewegung des Lagerelements und der Auflagefläche zueinander blockiert, nachdem die Auslöseeinheit den Lanzettenhalter durch die relative Drehbewegung des Lagerelements und der Auflagefläche zueinander in die zweite Position überführt hat, so dass eine Wiederverwendung der Stechhilfe verhindert wird.

Bezüglich des Aufbaus der Stechhilfe und ihrer Funktionsweise mit Ausnahme des Wiederverwendungsschutzes wird ausdrücklich auf das Dokument EP 1 371 329 A1 verwiesen.

Erfindungsgemäß weist die Stechhilfe zusätzlich ein Blockierelement auf, das nach dem Stechvorgang eine relative Drehbewegung des Lagerelements und der Auflagefläche zueinander blockiert. Diese Blockierung verhindert, dass die Stechhilfe nach der ersten Verwendung wieder verwendet wird.

Vorzugsweise blockiert das Blockierelement eine relative Drehbewegung des Lagerelements und der Auflagefläche zueinander automatisch, nachdem die Auslöseeinheit den Lanzettenhalter durch die relative Drehbewegung des Lagerelements und der Auflagefläche zueinander in die zweite Position überführt hat. Es kann aber auch eine Handlung des Anwenders erforderlich sein, um ein Blockieren der Drehbewegung durch das Blockierelement zu erreichen.

Das Blockierelement ist so angeordnet, dass es nach dem Auslösen der Stechbewegung entweder eine Drehbewegung des Lagerelements relativ zu der Auflagefläche zurück in die erste Position oder eine erneute Drehbewegung des Lagerelements relativ zu der Auflagefläche aus der ersten in die zweite Position blockiert. In dem ersten Fall wird aufgrund der Blockierung durch das Blockierelement ein erneutes Spannen der Stechhilfe durch eine Drehung des Lagerelements zurück auf die Auflagefläche verhindert. Im zweiten Fall ist gegebenenfalls ein erneutes Spannen der Stechhilfe möglich, sie kann danach jedoch nicht mehr ausgelöst werden, da das Blockierelement verhindert, dass das Lagerelement erneut von der Auflagefläche weggedreht werden kann und von dieser herabfällt.

Gemäß einer bevorzugten Ausfixhrungsform der vorliegenden Erfindung führt die zu der Auslöseeinheit gehörende Auslösetaste bei ihrer Betätigung eine lineare Bewegung aus und die lineare Bewegung wird in eine relative Drehbewegung des Lagerelements und der Auflagefläche zueinander überführt. Dabei ist das Blockierelement so angeordnet, dass es eine relative Drehbewegung des Lagerelements und der Auflagefläche zueinander blockiert, nachdem die Auslösetaste die lineare Bewegung zum Auslösen einer Stechbewegung der Lanzette ausgeführt hat.

Gemäß einer besonders bevorzugten Ausführungsform der vorliegenden Erfindung enthält die Auslöseeinheit mindestens ein Drehantriebselement, das beim Auslösen des Stechvorgangs durch die lineare Bewegung der Auslösetaste gegen das mindestens eine Lagerelement gedrückt wird und dadurch eine Drehbewegung des Lagerelements relativ zu der Auflagefläche bewirkt, wobei die Auslösetaste mindestens einen Haken aufweist, der so angeordnet ist, dass er sich nach der linearen Bewegung der Auslösetaste in dem Gehäuse verhakt, so dass die Auslösetaste keine lineare Rückbewegung durchführen kann und das Drehantriebselement so angeordnet bleibt, dass es als Blockierelement eine Drehbewegung des Lagerelements relativ zu der Auflagefläche zurück in die erste Position blockiert. Das Drehantriebselement wird durch die lineare Bewegung der Auslösetaste gegen das Lagerelement gedrückt und verdrängt dieses von der Auflagefläche, so dass die Lanzette die Stechbewegung ausführt. Die Auslösetaste wird durch den Haken in einer gedrückten Position verhakt, so dass sie nicht durch eine lineare Rückbewegung in ihre Ausgangsposition vor dem Auslösen der Stechhilfe zurückkehren kann. Sie wird in einer Position verhakt, in der das Drehantriebselement weiterhin auf der Auflagefläche angeordnet ist, von der es das Lagerelement verdrängt hat, und daher eine Rückrotation des Lagerelements in die erste Position blockiert.

Der Lanzettenhalter weist gemäß einer bevorzugten Ausführungsform der vorliegenden Erfindung zwei Lagerelemente auf, die in der ersten Position jeweils auf einer Auflagefläche des Gehäuses aufliegen. In diesem Fall weist der Auslöseknopf vorzugsweise zwei Drehantriebselemente auf. Es ist jedoch eine beliebige Anzahl von Lagerelementen und zugehörigen Auflageflächen und Drehantriebselementen denkbar.

Gemäß einer bevorzugten Ausführungsform der vorliegenden Erfindung verhakt sich der Haken nach der linearen Bewegung der Auslösetaste in einer Ausnehmung des Gehäuses oder hinter einem Vorsprung des Gehäuses. Der Haken kann bei der linearen Bewegung der Auslösetaste zum Beispiel über einen rampenförmigen Vorsprung des Gehäuses gleiten und sich nach der linearen Bewegung hinter dem rampenförmigen Vorsprung verhaken. Der Haken kann ferner die Auslösetaste vor dem Auslösen der Stechbewegung der Lanzette in einer anderen Position mit dem Gehäuse verhaken, um vor dem Auslösen des Stechvorgangs ein Abnehmen der Auslösetaste von dem Gehäuse zu verhindern. Dazu können zum Beispiel zwei Vorsprünge in dem Gehäuse vorgesehen sein, die so angeordnet sind, dass der Haken vor dem Auslösen eines Stechvorgangs hinter dem ersten Vorsprung verhakt ist und die Auslösetaste daher nicht vom Gehäuse abnehmbar ist, und nach der linearen Bewegung der Auslösetaste hinter dem zweiten Vorsprung verhakt ist, so dass das Blockierelement (Drehantriebselement) die Auflagefläche blockiert. Die Auslösetaste kann zu diesem Zweck aber auch mindestens zwei Haken aufweisen, wobei mindestens ein Haken zum Verhaken der Auslösetaste in dem Gehäuse vor dem Auslösen der Stechbewegung und mindestens ein weiterer Haken zum Verhaken der Auslösetaste in dem Gehäuse nach der linearen Bewegung zum Verhindern der linearen Rückbewegung der Auslösetaste dient.

Gemäß einer weiteren Ausführungsform weist die Stechhilfe gemäß der vorliegenden Erfindung einen abnehmbaren Sterilschutz auf, der Vorsprünge aufweist, die so angeordnet sind, dass der Sterilschutz nach der Stechbewegung der Lanzette als Blockierelement in die Öffnung des Gehäuses der Stechhilfe einführbar ist und sich mit den Vorsprüngen in der Öffnung verhakt und die Öffnung des Gehäuses blockiert, so dass eine Drehbewegung des Lagerelements relativ zu der Auflagefläche durch den Sterilschutz blockiert wird.

Bei der Lieferung befindet sich die erfindungsgemäße Stechhilfe in einem gespannten Zustand und über die Lanzette ist ein Sterilschutz gestülpt, um die Sterilität der Lanzette vor ihrem Gebrauch zu gewährleisten und unbeabsichtigte Verletzungen durch die Lanzette zu vermeiden. Der Sterilschutz ist vor der Verwendung der Stechhilfe abnehmbar. Nach der Verwendung kann der Sterilschutz als Blockierelement dienen, indem er (vorzugsweise in umgekehrter Richtung als vor seiner Abnahme) über die Öffnung durch den Anwender wieder in das Gehäuse eingeführt wird, wobei er sich in der Öffnung mittels der Vorsprünge verhakt und dort eine Drehbewegung des Lagerelements relativ zu der Auflagefläche und damit eine Wiederverwendung der Stechhilfe verhindert.

Der Sterilschutz der erfindungsgemäßen Stechhilfe ist vorzugsweise so ausgebildet, dass er sich nicht dazu eignet, nach dem Abnehmen von der Lanzette durch die Öffnung, durch die die Lanzettenspitze beim Stechvorgang austritt, in das Gehäuse hinein und wieder herausbewegt zu werden, so dass sich der Sterilschutz nicht als Werkzeug zum erneuten Spannen der Stechhilfe nach einem Einstechvorgang eignet. Dazu kann der Sterilschutz mindestens einen Abschnitt umfassen, der einen gegenüber der Öffnung des Gehäuses vergrößerten Durchmesser aufweist und zumindest in einer Richtung ein Einführen des Sterilschutzes in die Öffnung oder ein Herausziehen eines in die Öffnung eingeführten Sterilschutzes verhindert. Im letzteren Fall kann der Abschnitt mit vergrößertem Durchmesser in der Öffnung einrasten, sobald er in die Öffnung eingeführt wird.

Gemäß einer weiteren bevorzugten Ausfixhrungsform der vorliegenden Erfindung umfasst die Stechhilfe als Blockierelement ein Weichenelement, das in einer Richtung beweglich ausgebildet ist, um den Lanzettenhalter bei einer relativen Drehbewegung des Lagerelements und der Auflagefläche zueinander passieren zu lassen und in einer anderen Richtung nur bis in eine Blockierposition beweglich ausgebildet ist, wobei das Weichenelement in der Blockerposition den Lanzettenhalter so blockiert, dass es eine Drehbewegung zurück in die erste Position verhindert. Das Weichenelement ist also in der einen Richtung wegbewegbar, insbesondere wegklappbar oder wegbiegbar, so dass es beim Auslösen des Stechvorgangs den Lanzettenhalter nicht wesentlich behindert, so dass dieser die Stechbewegung ausführen kann. In der anderen Richtung ist das Blockierelement bis in eine Blockierposition bewegbar, insbesondere stellt oder richtet es sich auf, und steht in dieser Position einer Rückrotation des Lanzettenhalters nach dem Stechvorgang in seine Ausgangsposition, die er vor dem Auslösen des Stechvorgangs eingenommen hat, entgegen. Es blockiert also eine Drehbewegung zum erneuten Spannen der Stechhilfe.

Das Weichenelement ist vorzugsweise am Rand der Auflagefläche oder an dem Lanzettenhalter angeordnet. Mit dem Rand der Auflagefläche ist derjenige Rand gemeint, über den sich das Lagerelement beim Drehen von der ersten in die zweite Position bewegt, unmittelbar bevor der Lanzettenhalter die Fallbewegung ausführt. Vorzugsweise befindet sich angrenzend an diesen Rand eine entlang der Öffnung in dem Gehäuse verlaufende Führungsnut, in der das Lagerelement während einer linearen Fallbewegung bei dem Stechvorgang geführt wird.

Wenn das Weichenelement an dem Lanzettenhalter angeordnet ist, wirkt es mit einem Vorsprung in dem Gehäuse zusammen, an dem es in der Blockierposition anliegt.

Die Erfindung wird anhand der Zeichnung nachstehend näher erläutert.

Es zeigt:
- Figur 1: die schematische Zusammenbauskizze einer erfindungsgemäßen Stechhilfe,
- Figur 2: eine Aufsicht auf den Lanzettenhalter einer erfindungsgemäßen Stechhilfe gemäß Figur 1 innerhalb des Gehäuses,
- Figur 3A: ausschnittsweise eine erste Ausfixhrungsform einer erfindungsgemäßen Stechhilfe vor der ersten Verwendung,
- Figur 3B: ausschnittsweise eine erfindungsgemäße Stechhilfe gemäß Figur 3A nach der ersten Verwendung,
- Figur 4A: eine zweite Ausführungsform einer erfindungsgemäßen Stechhilfe vor der ersten Verwendung,
- Figur 4B: ausschnittsweise eine erfindungsgemäße Stechhilfe gemäß Figur 4A nach der ersten Verwendung,
- Figur 5A: eine dritte Ausführungsform einer erfindungsgemäßen Stechhilfe vor der ersten Verwendung,
- Figur 5B: ausschnittsweise eine erfindungsgemäße Stechhilfe gemäß Figur 5A nach der ersten Verwendung,
- Figur 6A: eine vierte Ausführungsform einer erfindungsgemäßen Stechhilfe vor der ersten Verwendung,
- Figur 6B: ausschnittsweise einen Sterilschutz der Stechhilfe gemäß Figur 6A,
- Figur 6C: ausschnittsweise einen nach der Verwendung der Stechhilfe gemäß Figur 6A in die Öffnung des Gehäuses eingeführten Sterilschutz,
- Figur 7A: eine Draufsicht auf das Innere des Gehäuses einer fünften Ausführungsform einer erfindungsgemäßen Stechhilfe,
- Figur 7B: ausschnittsweise das Gehäuseinnere der erfindungsgemäßen Stechhilfe gemäß Figur 7A,
- Figur 8A: eine Draufsicht auf das Innere des Gehäuses einer sechsten Ausführungsform einer erfindungsgemäßen Stechhilfe und
- Figur 8B: ausschnittsweise das Gehäuseinnere der erfindungsgemäßen Stechhilfe gemäß Figur 8A.

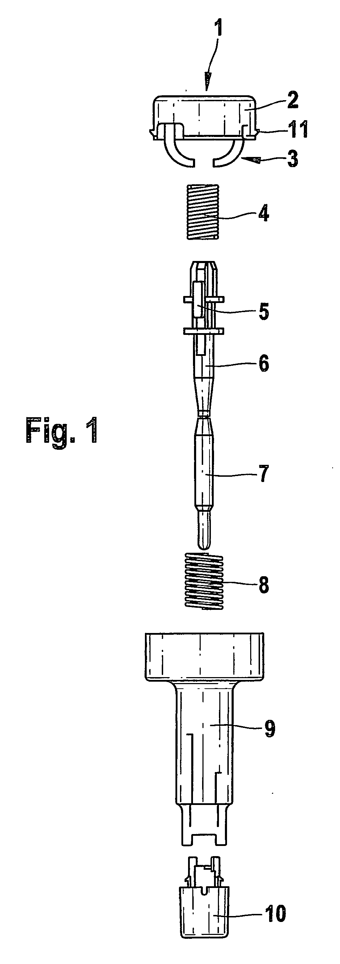

Figur 1 zeigt Bestandteile der erfindungsgemäßen Stechhilfe vor deren Zusammensetzung.

Die Stechhilfe verfügt über ein Gehäuse 9, in das der Lanzettenhalter 6 hineingeführt wird. Der Lanzettenhalter 6 wird im Gehäuse beweglich gelagert, so dass er bei einem Stechvorgang entlang der Einstichrichtung geführt werden kann. Hierfür beinhaltet das System weiterhin Antriebselemente, die mit dem Lanzettenhalter 6 verbunden sind und auf diese wirken. Die Antriebselemente werden im gezeigten Beispiel durch jeweils eine Feder verwirklicht (4 und 8). Hierbei kann im Sinne der Erfindung, wie in Figur 1 dargestellt, der Lanzettenhalter 6 lediglich lose auf einem Federelement aufliegen oder an diesem anliegen, ohne dass eine feste Verbindung zwischen Federelement und Lanzettenhalter 6 besteht. Im Sinne der Erfindung ist die Verbindung des Lanzettenhalters 6 mit einem Antriebselement dadurch charakterisiert, dass eine Kraft vom Antriebselement auf den Lanzettenhalter 6 übertragen werden kann. Es sind aber auch Ausführungsformen möglich, bei denen der Lanzettenhalter 6 fest mit einem Antriebselement verbunden ist. Der Lanzettenhalter 6 ist darüber hinaus derart im Gehäuse 9 positioniert, dass er während des Stechvorgangs senkrecht zur Einstichrichtung eine Rotation ausführen kann. Der Lanzettenhalter 6 selbst weist einen Sterilschutz 7 auf, der über eine Lanzette gestülpt ist, um eine Sterilität der Lanzette vor deren Gebrauch zu gewährleisten, sowie eventuelle unbeabsichtigte Verletzungen durch die Lanzettenspitze zu vermeiden. Zunächst wird die Feder 8 in das Gehäuse 9 eingeführt. Bei einem späteren Gebrauch der Stechhilfe wird somit ein Rücktransport der Lanzette in das Gehäuse nach dem Stechvorgang bewirkt. Die Feder 8 wird auch als Rückholfeder bezeichnet. Der Lanzettenhalter 6 wird innerhalb der Feder 8 sowie des Gehäuses eingeführt. Der Lanzettenhalter 6 verfügt weiterhin über Lagerelemente 5, die innerhalb des Gehäuses auf dafür vorgesehene Auflageflächen (nicht dargestellt) aufliegen. Sowohl die Feder 4 als auch die Feder 8 wirken in der Weise mit dem Lanzettenhalter 6 zusammen, dass beim Aufliegen der Lagerelemente 5 auf den Auflageflächen innerhalb des Gehäuses 9 die Feder 4 (Antriebsfeder) gespannt ist, wobei die Feder 8 (Rückholfeder) entspannt vorliegt. Während des Stechvorgangs entspannt sich die Antriebsfeder 4 unter Beschleunigung des Lanzettenhalters 6. Dieser führt eine Fallbewegung aus, wobei er auf die Rückholfeder 8, die dadurch gespannt wird, trifft. Der Lanzettenhalter 6 fällt innerhalb des Gehäuses bis zu einem Anschlag. Von der dann voll gespannten Rückholfeder 8 wird der Lanzettenhalter 6 wieder zurückgezogen. Zu diesem Zeitpunkt liegt die Antriebsfeder 4 entspannt vor. Am unteren Ende des Gehäuses 9 wird eine Kappe 10 beweglich montiert, so dass mittels einer Drehbewegung der Kappe die Länge desjenigen Teils der Lanzettenspitze, der aus dem Gehäuse austritt, verändert werden kann. Aufgrund dieses veränderbaren Abstandes der Kappe 10 können unterschiedliche Einstichtiefen der Lanzette eingestellt werden. Am oberen Ende des Gehäuses verschließt die Auslöseeinheit 1 die obere Gehäuseöffnung. Im dargestellten Beispiel beinhaltet die Auslöseeinheit eine Auslösetaste 2 sowie zwei elastische Häkchen 3 als Drehantriebselemente. Wird die Auslösetaste 2 entlang der Einstichrichtung der Lanzette betätigt, verursachen die Häkchen 3 eine Drehbewegung des Lanzettenhalters 6.

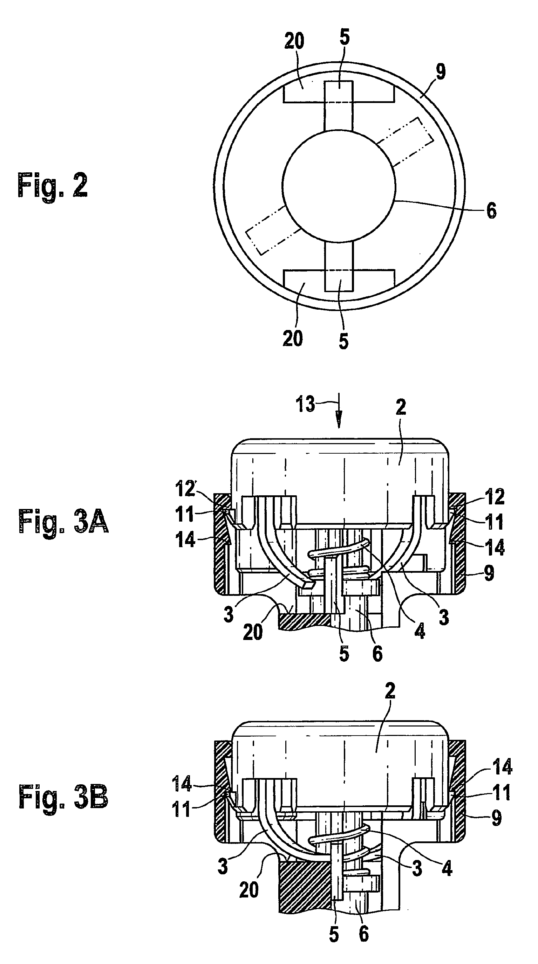

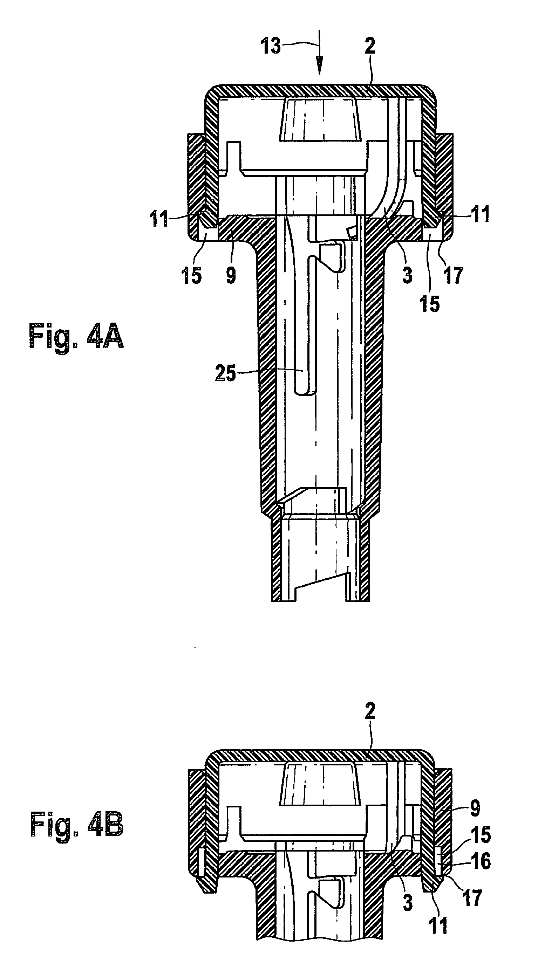

Die Auslösetaste 2 weist zwei Haken 11 auf, mit denen sich die Auslösetaste 2 in dem Gehäuse 9 verhakt, so dass sie im zusammengesetzten Zustand der Stechhilfe nicht von dieser abfällt oder abgenommen werden kann. Diese Haken können sich gemäß einer Ausführungsform der vorliegenden Erfindung nach dem Drücken der Auslösetaste 2 zum Auslösen eines Stechvorgangs (nach der linearen Bewegung der Auslösetaste 2) in einer eingedrückten Position der Auslösetaste 2 mit dem Gehäuse verhaken, so dass die Auslösetaste 2 keine lineare Rückbewegung in die Ausgangsposition durchführen kann und die Drehantriebselemente 3 so angeordnet bleiben, dass sie als Blockierelemente eine Drehbewegung der Lagerelemente 5 relativ zu den Auflageflächen 20 zurück in die erste Position blockieren. Diese besondere Ausführungsform wird in Bezug auf Figuren 3A, 3B, 4A und 4B näher erläutert.

Figur 2 zeigt eine Draufsicht auf die Stechhilfe gemäß Figur 1, bei der sich der Lanzettenhalter 6 in dem Lanzettengehäuse 9 in einer ersten Position befindet. Der Lanzettenhalter 6 verfügt in dem dargestellten Beispiel über zwei Lagerelemente 5, die in Form von Haltearmen ausgebildet sind. Die Haltearme 5 liegen in der ersten Position auf den Auflageflächen 20 des Gehäuses 9 auf. Durch die eine Rotationsbewegung des Lanzettenhalters 6 werden die Lagerelemente 5 in eine zweite Position (strichzweipunktiert dargestellt) bewegt, in der sie nicht mehr auf den Auflageflächen aufliegen. Der mit den Lagerelementen verbundene Lanzettenhalter kann nun eine Bewegung in Einstichrichtung ausführen. Durch die Bewegung des Lanzettenhalters in Einstichrichtung wird ein Stechvorgang ausgeführt.

Figur 3A zeigt ausschnittsweise eine erste Ausführungsform einer erfindungsgemäßen Stechhilfe vor der ersten Verwendung.

Es ist die Auslösetaste 2 dargestellt, die zwei Haken 11 aufweist. Die Haken 11 sind vor der Verwendung der Stechhilfe hinter zwei oberen Vorsprüngen 12 des Gehäuses 9 eingehakt, so dass die Auslösetaste 2 in das Gehäuse 9 oberhalb von unteren Vorsprüngen 14 des Gehäuses 9 eingerastet ist. Mit der Auslösetaste 2 sind Häkchen 3 als Drehantriebselemente verbunden. Die Häkchen 3 sind in der dargestellten (nicht gedrückten) Position der Auslösetaste 2 beabstandet zu den Lagerelementen 5 des Lanzettenhalters 6 angeordnet, die in der ersten Position auf den (nicht dargestellten) Auflageflächen 20 aufliegen. Die Antriebsfeder 4 befindet sich in einem gespannten (komprimierten) Zustand. Beim Drücken der Auslösetaste 2 in axialer Richtung 13 führt die Auslösetaste 2 eine lineare Bewegung in axialer Richtung aus. Dadurch werden die Drehantriebselemente 3 gegen die Lagerelemente 5 gedrückt, die folglich eine Drehbewegung relativ zu den Auflageflächen 20 ausführen. Der Lanzettenhalter 6 wird dadurch gedreht, bis die Lagerelemente 5 von den Auflageflächen 20 fallen. Der Lanzettenhalter 6 inklusive Lanzette wird dann durch die Antriebsfeder 4 in axialer Richtung 13 beschleunigt und führt eine Stechbewegung aus.

Figur 3B zeigt ausschnittsweise eine erfindungsgemäße Stechhilfe gemäß Figur 3A nach der ersten Verwendung.

Durch das Drücken der Auslösetaste 2 wurde diese in dem Gehäuse 9 versenkt. Die Haken 11 haben sich dabei in dem Gehäuse 9 hinter den unteren Vorsprüngen 14 verhakt, so dass die Auslösetaste 2 keine lineare Rückbewegung in ihre Ausgangsposition gemäß Figur 3A ausführen kann. In der in Figur 3B dargestellten verhakten Position der Auslösetaste 2 liegen die Drehantriebselemente 3 auf den Auflageflächen 20 auf, von denen sie beim Auslösen der Stechhilfe die Lagerelemente 5 verdrängt haben. Die Drehantriebselemente 3 dienen daher als Blockierelemente, die eine Drehbewegung der Lagerelemente 5 auf die Auflageflächen 20 verhindern.

Bei der in den Figuren 3A und 3B dargestellten Ausführungsform einer erfindungsgemäßen Stechhilfe sind die unteren Vorsprünge 14 als rampenförmige Vorsprünge ausgebildet, über die die Haken 11 bei der linearen Bewegung der Auslösetaste 2 in axialer Richtung 13 gleiten, bis sie sich hinter den unteren Vorsprüngen 14 verhaken, wie in Figur 3B gezeigt.

Figur 4A zeigt eine zweite Ausführungsform einer erfindungsgemäßen Stechhilfe vor der ersten Verwendung.

Die Auslösetaste 2 dieser Stechhilfe weist zwei Haken 11 auf, die die Auslösetaste 2 in jeweils einer Ausnehmung 15 des Gehäuses 9 verhaken, damit die Auslösetaste 2 nicht von dem Gehäuse 9 abgenommen werden kann. In der in Figur 4A dargestellten Stechhilfe liegen die Lagerelemente 5 des Lanzettenhalters 6 auf den Auflageflächen 20 auf (nicht dargestellt), wobei die Drehantriebselemente 3 (Häkchen) von den Lagerelementen 5 beabstandet angeordnet sind.

Beim Drücken der Auslösetaste 2 zum Auslösen eines Stechvorgangs werden die Lagerelemente 5 durch die Drehantriebselemente 3 von den Auflageflächen 20 verdrängt und fallen in die Führungsnuten 25. Die Haken 11 gleiten dabei jeweils durch eine Gehäuseöffnung 16, bis sie aus dem Gehäuse 9 austreten und sich außen an dem Gehäuse 9 hinter einem Rand 17 verhaken. Diese verhakte Position ist in Figur 4B dargestellt. Dabei sind die Drehantriebselemente 3 auf den Auflageflächen 20 positioniert (nicht dargestellt), so dass sie als Blockierelemente dienen, die eine Drehung der Lagerelemente zurück auf die Auflageflächen nicht erlauben.

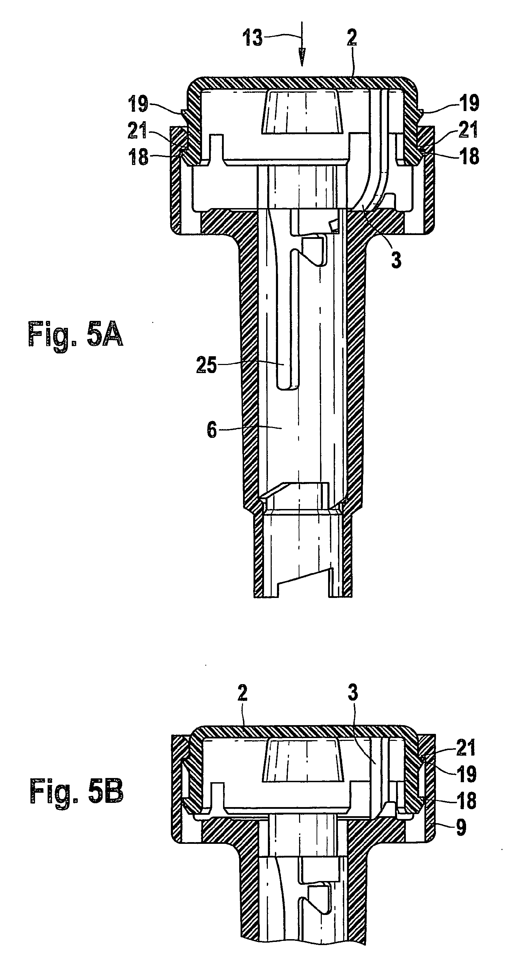

Figur 5A zeigt eine dritte Ausführungsform einer unbenutzten erfindungsgemäßen Stechhilfe.

Die Auslösetaste 2 dieser Stechhilfe weist 4 Haken auf, zwei untere Haken 18 und zwei obere Haken 19. In der in Figur 5A dargestellten Position sind die unteren Haken 18 hinter Vorsprüngen 21 am oberen Rand des Gehäuses 9 verhakt, so dass die Auslösetaste 2 an dem Gehäuse festgehalten wird. Die als Häkchen ausgebildeten Drehantriebselemente 3 sind beabstandet zu den Lagerelementen 5 angeordnet, die auf den Auflageflächen 20 aufliegen (nicht dargestellt).

Durch ein Drücken der Auslösetaste 2 wird diese in axialer Richtung 13 linear verschoben. Dabei werden die Drehantriebselemente 3 gegen die Lagerelemente 5 gedrückt, so dass der Lanzettenhalter 6 eine Drehbewegung ausführt und die Lagerelemente 5 von den (nicht dargestellten) Auflageflächen 20 in die Führungsnuten 25 fallen. Die Lanzette führt dann eine Stechbewegung aus. Die Auslösetaste 2 ist, wie in Figur 5B gezeigt, nach der linearen Bewegung in dem Gehäuse 9 mit dem oberen Haken 19 hinter den Vorsprüngen 21 verhakt, um eine lineare Rückbewegung der Auslösetaste 2 in ihre Ausgangsposition zu verhindern. In dieser Position blockieren die Drehantriebselemente 3 (Blockierelemente) die Auflageflächen 20, so dass eine Rückdrehung der Lagerelemente 5 auf die Auflageflächen 20 verhindert wird. Die Stechhilfe ist daher nicht wieder verwendbar.

Für die unteren Haken 18 und die oberen Haken 19 können auch verschiedene Vorsprünge in dem Gehäuse 9 vorgesehen sein, wobei die Haken 18, 19 dann vorzugsweise nicht direkt übereinander an der Auslösetaste 2, sondern entlang des Umfangs der Auslösetaste 2 verschoben an der Außenseite der Auslösetaste 2 angeordnet sind.

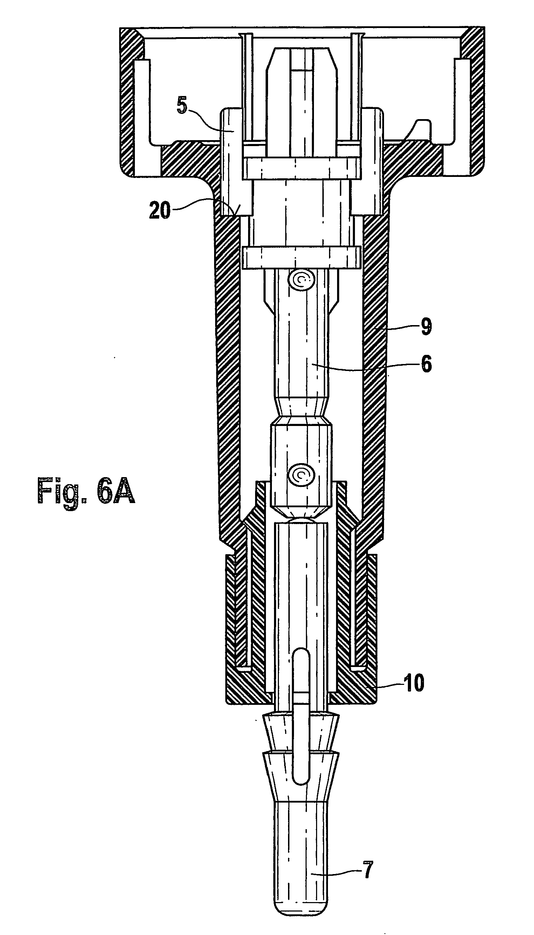

Figur 6A zeigt eine vierte Ausführungsform einer erfindungsgemäßen Stechhilfe vor der ersten Verwendung.

Die Stechhilfe weist einen als Blockierelement verwendbaren Sterilschutz 7 auf, der vor der ersten Verwendung der Stechhilfe abnehmbar über eine Lanzette gestülpt ist und mit einem Ende an dem Lanzettenhalter 6 anliegt. Ansonsten ist die Stechhilfe vorzugsweise wie in EP 1 371 329 A1 beschrieben, aufgebaut und umfasst eine Auslösetaste 2, ein Gehäuse 9 mit Auflagefläche 20 und einen Lanzettenhalter 6 mit Lagerelement 5.

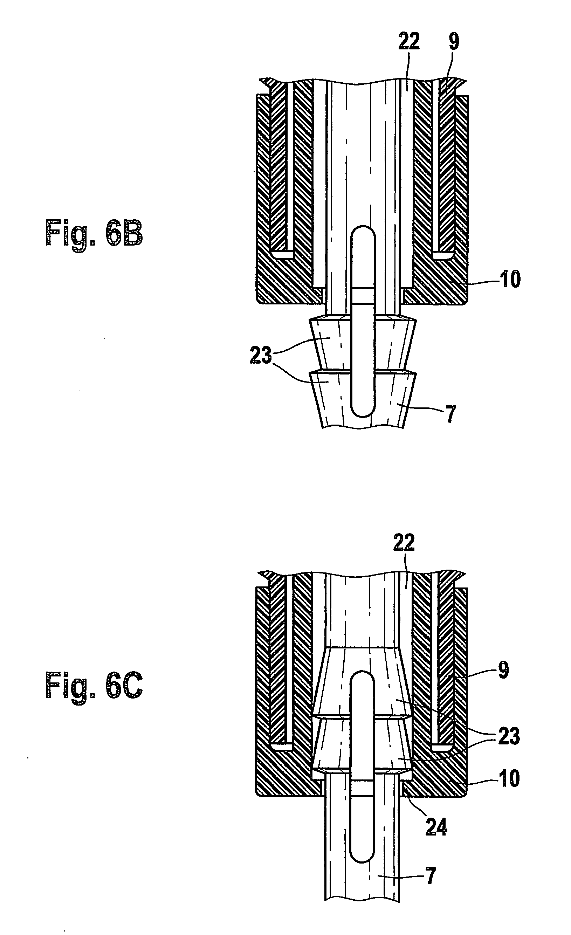

In Figur 6B ist vergrößert dargestellt, wie der Sterilschutz im unbenutzten Zustand der Stechhilfe aus dem Gehäuse herausragt.

Der Sterilschutz 7 steckt in der Öffnung 22 des Gehäuses 9. Die Kappe 10 zur Einstellung der Einstechtiefe umgibt das Gehäuse 9 im dargestellten Bereich. Der Sterilschutz 7 weist ringförmige Vorsprünge 23 auf, die vor der Verwendung der Stechhilfe außerhalb der Öffnung 22 des Gehäuses 9 positioniert sind. Zur Verwendung der Stechhilfe wird der Sterilschutz 7 von der Lanzettenspitze ab- und aus der Öffnung 22 herausgezogen.

Nach der Verwendung der Stechhilfe kann der Sterilschutz 7 als Blockierelement dienen. Dazu wird der Sterilschutz 7 umgedreht und in umgekehrter Richtung wieder in die Öffnung 22 des Gehäuses 9 hereingeschoben. Dabei rasten die Vorsprünge 24 des Gehäuses 9 hinter den ringförmigen Vorsprüngen 23 des Sterilschutzes 7 ein, so dass sich der Sterilschutz 7 nicht mehr aus der Öffnung 22 des Gehäuses 9 herausziehen lässt, wie in Figur 6C dargestellt. Der Sterilschutz verhindert in dieser Position, dass das Lagerelement 5 und die Auflagefläche 20 eine relative Drehbewegung zueinander ausführen, da kein Werkzeug mehr von außen in die Öffnung 22 des Gehäuses 9 eingeführt werden kann, das eine solche Drehbewegung verursachen könnte. Die Stechhilfe ist somit vor einer Wiederverwendung geschützt.

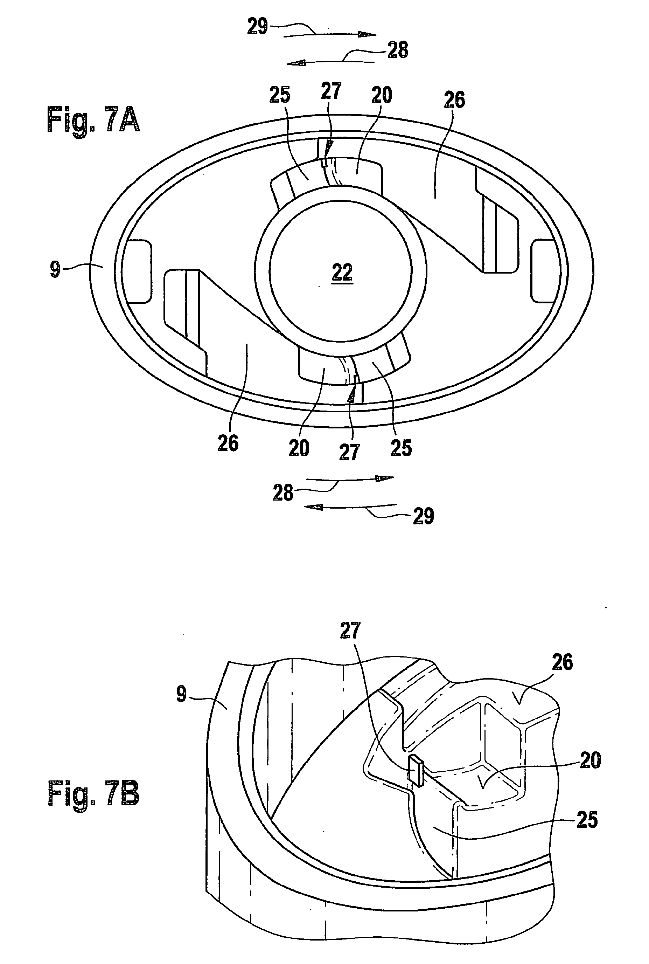

Figur 7A zeigt eine Draufsicht auf das Innere des Gehäuses einer fünften Ausführungsform einer erfindungsgemäßen Stechhilfe.

In dem Gehäuse 9 sind zwei Auflageflächen 20 für zwei Lagerelemente 5 sichtbar. Ferner ist das obere Ende zweier Führungsnuten 25 neben den Auflageflächen 20 angeordnet, die entlang der Öffnung 22 des Gehäuses 9 verlaufen. Wenn die Lagerelemente 5 (nicht dargestellt) in die zweite Position gedreht werden, fallen sie in diese Führungsnuten 25, durch die sie während der Fallbewegung gleiten.

Weiterhin enthält das Gehäuse 9 Führungsflächen 26, die zum Führen der Drehantriebselemente der Auslösetaste dienen, wenn die Auslösetaste zum Auslösen des Stechvorgangs gedrückt wird. Die Drehantriebselemente gleiten dann entlang dieser Führungsflächen 26 und drücken gegen die Lagerelemente, wodurch eine Drehbewegung der Lagerelemente relativ zu den Auflageflächen 20 verursacht wird. Am Rand der Auflageflächen 20 sind als Blockierelemente Weichenelemente 27 vorgesehen. Diese Weichenelemente 27 sind in einer ersten Richtung 28 beweglich ausgebildet, um das jeweilige Lagerelement des Lanzettenhalters bei einer relativen Drehbewegung des Lagerelements und der Auflagefläche 20 in die zweite Position des Lanzettenhalters passieren zu lassen. Beweglich ausgebildet bedeutet dabei, dass das Weichenelement 27 in dieser Richtung weggeklappt oder weggebogen werden kann. In einer zweiten Richtung 29 sind die Weichenelemente 27 nur bis in eine Blockierposition beweglich, in der das jeweilige Weichenelement 27 den Lanzettenhalter (beziehungsweise die Lagerelemente) so blockiert, dass es eine Drehbewegung zurück in die erste Position verhindert. Die Weichenelemente 27 dienen folglich als Blockierelemente. In der Blockierposition sind die Weichenelemente 27 zum Beispiel relativ zu den Auflageflächen 20 senkrecht aufgerichtet. Sie können jedoch auch jede andere Position einnehmen, in der sie die Rückbewegung des Lagerelements in die erste Position auf die Auflagefläche blockieren.

Figur 7B zeigt perspektivisch einen Ausschnitt aus dem Gehäuseinneren der erfindungsgemäßen Stechhilfe gemäß Figur 7A.

Es sind insbesondere eine Auflagefläche 20, ein Weichenelement 27, eine Führungsnut 25 und eine Führungsfläche 26 in dem Gehäuse 9 erkennbar.

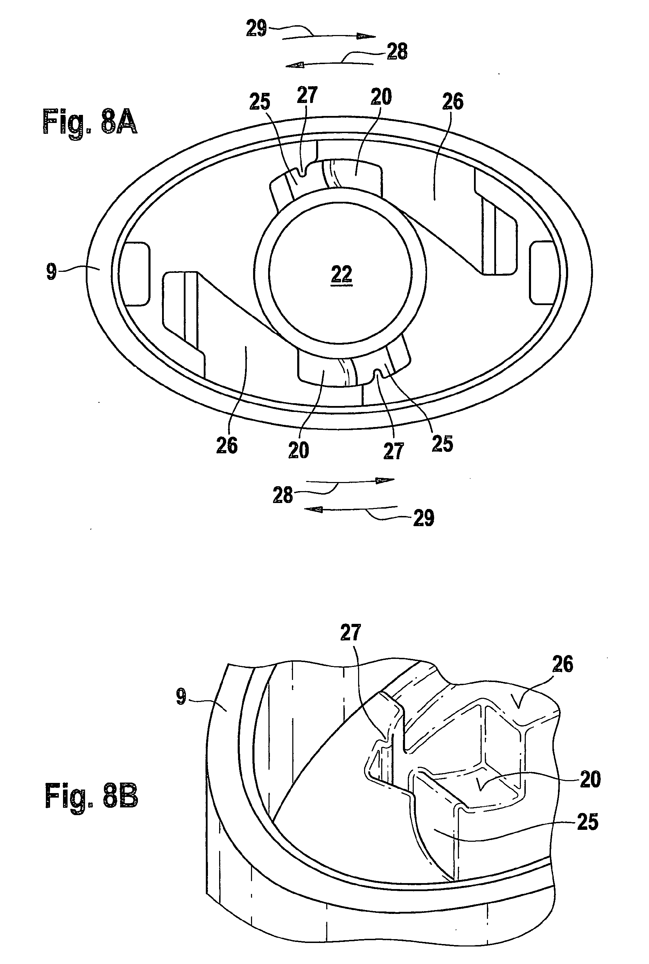

Figur 8A zeigt eine Draufsicht auf das Innere des Gehäuses einer sechsten Ausführungsform einer erfindungsgemäßen Stechhilfe.

Diese erfindungsgemäße Stechhilfe weist ein Gehäuse 9 mit zwei Führungsflächen 26, zwei Führungsnuten 25, zwei Auflageflächen 20 und zwei Weichenelementen 27 auf. Die Funktionsweise der erfindungsgemäßen Stechhilfe entspricht der bezüglich Figuren 7A und 7B beschriebenen. Dabei sind die als Blockierelemente vorgesehenen Weichenelemente 27 an anderer Stelle im Gehäuse 9 angebracht. Sie befinden sich jeweils am Rand einer Führungsnut 25 neben der Auflagefläche 20.

Figur 8B zeigt perspektivisch einen Ausschnitt aus dem Gehäuseinneren der erfindungsgemäßen Stechhilfe gemäß Figur 8B.

Dargestellt sind insbesondere eine Führungsfläche 26, eine Auflagefläche 20, ein Weichenelement 27 und eine Führungsnut 25 in dem Gehäuse 9.

### Bezugszeichenliste

- 1: Auslöseeinheit
- 2: Auslösetaste
- 3: Häkchen, Drehantriebselemente
- 4: Antriebsfeder
- 5: Lagerelemente
- 6: Lanzettenhalter
- 7: Sterilschutz
- 8: Rückholfeder
- 9: Gehäuse
- 10: Kappe
- 11: Haken
- 12: obere Vorsprünge
- 13: axiale Richtung
- 14: untere Vorsprünge
- 15: Ausnehmungen
- 16: Gehäuseöffnungen
- 17: Rand
- 18: untere Haken
- 19: obere Haken
- 20: Auflageflächen
- 21: Vorsprünge
- 22: Öffnung
- 23: ringförmige Vorsprünge
- 24: Vorsprünge
- 25: Führungsnuten
- 26: Führungsflächen
- 27: Weichenelemente
- 28: erste Richtung
- 29: zweite Richtung

## Patentansprüche

1. Stechhilfe umfassend
- ein Gehäuse (9) mit einer Öffnung (22), in dem ein Lanzettenhalter (6) mit einer Lanzette beweglich gelagert ist, wobei der Lanzettenhalter (6) mit einem Federelement verbunden ist und der Lanzettenhalter (6) mindestens ein Lagerelement (5) umfasst, und in dem Gehäuse (9) mindestens eine Auflagefläche (20) in der Weise angeordnet ist, dass das Lagerelement (5) in einer ersten Position des Lanzettenhalters (6) auf der Auflagefläche (20) aufliegt, sowie
- eine Auslöseeinheit (1), durch die der Lanzettenhalter (6) durch Betätigen einer Auslösetaste (2) mittels einer relativen Drehbewegung des Lagerelements (5) und der Auflagefläche (20) zueinander in eine zweite Position überführt wird, so dass das Lagerelement (5) in der zweiten Position des Lanzettenhalters (6) von der Auflagefläche (20) fällt, wobei sich das vor der Fallbewegung gespannte Federelement zumindest teilweise entspannt und den Lanzettenhalter (6) relativ zum Gehäuse (9) bewegt, so dass die Spitze der Lanzette aus der Öffnung (22) des Gehäuses (9) austritt,
**dadurch gekennzeichnet,**
**dass** die Stechhilfe ein Blockierelement umfasst, das so angeordnet ist, dass es eine relative Drehbewegung des Lagerelements (5) und der Auflagefläche (20) zueinander blockiert, nachdem die Auslöseeinheit (1) den Lanzettenhalter (6) durch die relative Drehbewegung des Lagerelements (5) und der Auflagefläche (20) zueinander in die zweite Position überführt hat, so dass eine Wiederverwendung der Stechhilfe verhindert wird, wobei das Blockierelement so angeordnet ist, dass es nach dem Auslösen der Stechbewegung eine Drehbewegung des Lagerelements (5) relativ zu der Auflagefläche (20) zurück in die erste Position oder eine erneute Drehbewegung des Lagerelements (5) relativ zu der Auflagefläche (20) aus der ersten in die zweite Position, wobei das Lagerelement (5) erneut von der Auflagefläche (20) herabfällt, blockiert.

2. Stechhilfe gemäß Anspruch 1, **dadurch gekennzeichnet, dass** die Auslösetaste (2) der Auslöseeinheit (1) bei Betätigung eine lineare Bewegung ausführt und die lineare Bewegung in eine relative Drehbewegung des Lagerelements (5) und der Auflagefläche (20) zueinander überführt wird und das Blockierelement so angeordnet ist, dass es eine relative Drehbewegung des Lagerelements (5) und der Auflagefläche (20) zueinander blockiert, nachdem die Auslösetaste (2) die lineare Bewegung zum Auslösen einer Stechbewegung der Lanzette ausgeführt hat.

3. Stechhilfe gemäß einem der Ansprüche 1 oder 2, **dadurch gekennzeichnet, dass** das Blockierelement eine relative Drehbewegung des Lagerelements (5) und der Auflagefläche (20) zueinander automatisch blockiert, nachdem die Auslöseeinheit (1) den Lanzettenhalter (6) durch die relative Drehbewegung des Lagerelements (5) und der Auflagefläche (20) zueinander in die zweite Position überführt hat.

4. Stechhilfe gemäß einem der Ansprüche 1 bis 3, **dadurch gekennzeichnet, dass** die Auslöseeinheit (1) mindestens ein Drehantriebselement (3) enthält, das durch eine lineare Bewegung der Auslösetaste (2) gegen das mindestens eine Lagerelement (5) gedrückt wird und dadurch eine Drehbewegung des Lagerelements (5) relativ zu der Auflagefläche (20) bewirkt, wobei die Auslösetaste (2) mindestens einen Haken (11, 19) aufweist, der so angeordnet ist, dass er sich nach der linearen Bewegung der Auslösetaste (2) in dem Gehäuse (9) verhakt, so dass die Auslösetaste (2) keine lineare Rückbewegung durchführen kann und das Drehantriebselement (3) so angeordnet bleibt, dass es als Blockierelement eine Drehbewegung des Lagerelements (5) relativ zu der Auflagefläche (20) zurück in die erste Position blockiert.

5. Stechhilfe gemäß Anspruch 4, **dadurch gekennzeichnet, dass** sich der Haken (11, 19) nach der linearen Bewegung der Auslösetaste (2) in einer Ausnehmung des Gehäuses oder hinter einem Vorsprung (14, 21) des Gehäuses (9) verhakt.

6. Stechhilfe gemäß einem der Ansprüche 4 oder 5, **dadurch gekennzeichnet, dass** der Haken (11, 19) bei der linearen Bewegung der Auslösetaste (2) über einen rampenförmigen Vorsprung (14) des Gehäuses (9) gleitet und sich nach der linearen Bewegung hinter dem rampenförmigen Vorsprung (14) verhakt.

7. Stechhilfe gemäß einem der Ansprüche 4 bis 6, **dadurch gekennzeichnet, dass** der Haken (11, 19) die Auslösetaste (2) vor dem Auslösen der Stechbewegung der Lanzette mit dem Gehäuse (9) verhakt, um ein Abnehmen der Auslösetaste (2) von dem Gehäuse (9) zu verhindern.

8. Stechhilfe gemäß einem der Ansprüche 4 bis 7, **dadurch gekennzeichnet, dass** die Auslösetaste (2) mindestens zwei Haken (18, 19) aufweist, wobei mindestens ein Haken (18) zum Verhaken der Auslösetaste (2) in dem Gehäuse (9) vor dem Auslösen der Stechbewegung und mindestens ein weiterer Haken (19) zum Verhaken der Auslösetaste (2) in dem Gehäuse (9) nach der linearen Bewegung zum Verhindern der linearen Rückbewegung der Auslösetaste (2) dient.

9. Stechhilfe gemäß einem der Ansprüche 1 bis 8, **dadurch gekennzeichnet, dass** die Stechhilfe einen abnehmbaren Sterilschutz (7) aufweist, der Vorsprünge (23) aufweist, die so angeordnet sind, dass der Sterilschutz (7) nach der Stechbewegung der Lanzette als ein Blockierelement in die Öffnung (22) des Gehäuses (9) einführbar ist und sich mit seinen Vorsprüngen (23) in der Öffnung (22) verhakt und die Öffnung (22) des Gehäuses (9) blockiert, so dass eine Drehbewegung des Lagerelements (5) relativ zu der Auflagefläche (20) durch den Sterilschutz (7) verhindert wird.

10. Stechhilfe gemäß einem der Ansprüche 1 bis 9, **dadurch gekennzeichnet, dass** die Stechhilfe als Blockierelement ein Weichenelement (27) umfasst, das in einer Richtung beweglich ausgebildet ist, um den Lanzettenhalter (6) bei einer relativen Drehbewegung des Lagerelements (5) und der Auflagefläche (20) zueinander passieren zu lassen und in einer anderen Richtung nur bis in eine Blockierposition beweglich ausgebildet ist, wobei das Weichenelement (27) in der Blockierposition den Lanzettenhalter (6) so blockiert, dass es eine Drehbewegung zurück in die erste Position verhindert.

11. Stechhilfe gemäß Anspruch 10, **dadurch gekennzeichnet, dass** das Weichenelement (27) am Rand der Auflagefläche (20) oder an dem Lanzettenhalter (6) angeordnet ist.

## Claims

1. Puncturing auxiliary device comprising
- a housing (9) with an aperture (22), in which a lancet holder (6) is movably mounted with a lancet, the lancet holder (6) being connected to a spring element and the lancet holder (6) comprising at least a bearing element (5), and at least a bearing surface (20) being arranged in the housing (9) such that the bearing element (5) is located on the bearing surface (20) in a first position of the lancet holder (6) and
- a release mechanism (1), by which the lancet holder (6) is transferred to a second position by actuating a release button (2) by means of a rotating movement of the bearing element (5) and the bearing surface (20) relative to each other, so that the bearing element (5) in the second position of the lancet holder (6) drops from the bearing surface (20), the spring element having been tensioned prior to the drop movement at least partially relaxing and moving the lancet holder (6) relative to the housing, so that the lancet tip protrudes out of the aperture (22) in the housing (9),
**characterised in that**
the puncturing auxiliary device comprises a blocking element, which is arranged so that it blocks a rotating movement of the bearing element (5) and the bearing surface (20) relative to each other, after the release mechanism (1) has transferred the lancet holder (6) into the second position by the rotating movement of the bearing element (5) and the bearing surface (20) relative to each other, so that the puncturing auxiliary device cannot be reused, the blocking element being arranged so that after triggering the lancing movement it blocks a rotating movement of the bearing element (5) relative to the bearing surface (20) back to the first position or a renewed rotating movement of the bearing element (5) relative to the bearing surface (20) from the first into the second position, the bearing element (5) dropping again from the bearing surface (20).

2. Puncturing auxiliary device according to claim 1, **characterised in that** the release button (2) in the release mechanism (1) carries out a linear movement when actuated and the linear movement is transferred by a rotating movement of the bearing element (5) and the bearing surface (20) relative to each other and the blocking element is arranged so that it blocks a rotating movement of the bearing element (5) and the bearing surface (20) relative to each other, after the release button (2) has carried out the linear movement to trigger a lancing movement in the lancet.

3. Puncturing auxiliary device according to any of claims 1 or 2, **characterised in that** the blocking element automatically blocks a rotating movement of the bearing element (5) and the bearing surface (20) relative to each other, after the release mechanism (1) has transferred the lancet holder (6) into the second position by the rotating movement of the bearing element (5) and the bearing surface (20) relative to each other.

4. Puncturing auxiliary device according to any of claims 1 to 3, **characterised in that** the release mechanism (1) includes at least a rotating drive element (3), which is pressed against at least a bearing element (5) via a linear movement of the release button (2) and thereby causes a rotating movement in the bearing element (5) relative to the bearing surface (20), the release button (2) displaying at least a catch (11, 19), which is arranged so that it is caught in the housing (9) after the linear movement of the release button (2), so that the release button (2) cannot carry out any linear return movement and the rotating drive element (3) remains arranged so that as a blocking element it blocks a rotating movement in the bearing element (5) relative to the bearing surface (20) back to the first position.

5. Puncturing auxiliary device according to claim 4, **characterised in that** the catch (11,19) is caught in a recess in the housing or behind a projection (14,21) in the housing (9) after the linear movement of the release button (2).

6. Puncturing auxiliary device according to any of claims 4 or 5, **characterised in that**, through the linear movement of the release button (2), the catch (11,19) slides over a wedge-shaped projection (14) in the housing (9) and is caught behind the wedge-shaped projection (14) after the linear movement.

7. Puncturing auxiliary device according to any of claims 4 to 6, **characterised in that** the release button (2) is caught in the housing (9) by the catch (11,19) before the lancing movement of the lancet is triggered in order to prevent the release button (2) being removed from the housing (9).

8. Puncturing auxiliary device according to any of claims 4 to 7, **characterised in that** the release button (2) displays at least two catches (18,19), at least a catch (18) being used to catch the release button (2) in the housing (9) before the lancing movement is triggered and at least a further catch (19) being used to catch the release button (2) in the housing (9) after the linear movement to prevent the linear return movement of the release button (2).

9. Puncturing auxiliary device according to any of claims 1 to 8, **characterised in that** the puncturing auxiliary device has a removable sterile protective cover (7), which has projections (23) that are arranged so that the sterile protective cover (7) can be introduced into the aperture (22) of the housing (9) as a blocking element after the lancing movement of the lancet and is caught in the aperture (22) by its projections (23) which it uses to block the aperture (22) in the housing (9), so that a rotating movement of the bearing element (5) relative to the bearing surface (20) is prevented by the sterile protective cover (7).

10. Puncturing auxiliary device according to any of claims 1 to 9, **characterised in that** as a blocking element the puncturing auxiliary device comprises a switch element (27) that is designed to move in a direction to allow the lancet holder (6) to pass during a rotating movement of the bearing element (5) and the bearing surface (20) relative to each other and is designed to move in another direction only up to a blocking position, the switch element (27) blocking the lancet holder (6) in the blocking position so that it prevents a rotating movement back to the first position.

11. Puncturing auxiliary device according to claim 10, **characterised in that** the switch element (27) is arranged at the edge of the bearing surface (20) or on the lancet holder (6).

## Revendications

1. Auxiliaire de piqûre comprenant
- un logement (9) comportant une ouverture (22), dans lequel est monté en mobilité un dispositif de support de lancettes (6) comprenant une lancette, le dispositif de support de lancettes (6) étant relié à un élément faisant ressort et le dispositif de support de lancettes (6) comprenant au moins un élément de palier (5), et au moins une surface d'appui (20) étant disposée dans le logement de telle sorte que l'élément de palier (5) vient s'appuyer, dans une première position du dispositif de support de lancettes (6), contre la surface d'appui (20), et
- une unité de déclenchement (1) par laquelle le dispositif de support de lancettes (6), via l'actionnement d'une touche de déclenchement (2), au moyen d'un mouvement rotatif relatif de l'élément de palier (5) et de la surface d'appui (20) l'un par rapport à l'autre, est transféré dans une deuxième position, si bien que l'élément de palier 5 dans la deuxième position du dispositif de support de lancettes (6) s'écarte de la surface d'appui (20) en tombant, donnant lieu au fait que l'élément de ressort, tendu avant le mouvement de chute, se détend au moins en partie et déplace le dispositif de support de lancettes (6) par rapport au logement (9), si bien que la pointe de la lancette sort de l'ouverture (22) du logement (9),
**caractérisé en ce que**
l'auxiliaire de piqûre comprend un élément de blocage qui est disposé de telle sorte qu'il bloque un mouvement rotatif relatif de l'élément de palier (5) et de la surface d'appui (20) l'un par rapport à l'autre, après le transfert, par l'unité de déclenchement (1), du dispositif de support de lancettes (6) dans la deuxième position via le mouvement rotatif relatif de l'élément de palier (5) et de la surface d'appui (20) l'un par rapport à l'autre, si bien qu'une réutilisation de l'auxiliaire de piqûre est empêchée, l'élément de blocage étant disposé de telle sorte qu'après le déclenchement du mouvement de piqûre, il bloque un mouvement rotatif de l'élément de palier (5) par rapport à la surface d'appui (20) en retour dans la première position ou un mouvement rotatif renouvelé de l'élément de palier (5) par rapport à la surface d'appui (20) de la première à la deuxième position, mouvement au cours duquel l'élément de palier (5) s'écarte à nouveau de la surface d'appui (20) en tombant.

2. Auxiliaire de piqûre selon la revendication 1, **caractérisé en ce que** la touche de déclenchement (2) de l'unité de déclenchement (1) effectue un mouvement linéaire, lors de son actionnement, le mouvement linéaire se transformant en un mouvement rotatif relatif de l'élément de palier (5) et de la surface d'appui (20) l'un par rapport à l'autre, et l'élément de blocage est disposé de telle sorte qu'il bloque un mouvement rotatif relatif de l'élément de palier (5) et de la surface d'appui (20) l'un par rapport à l'autre, après que la touche de déclenchement (2) a effectué le mouvement linéaire pour le déclenchement d'un mouvement de piqûre de la lancette.

3. Auxiliaire de piqûre selon la revendication 1 ou 2, **caractérisé en ce que** l'élément de blocage bloque automatiquement un mouvement rotatif relatif de l'élément de palier (5) et de la surface d'appui (20) l'un par rapport à l'autre, après le transfert du dispositif de support de lancettes (6) dans la deuxième position par l'unité de déclenchement (1) via le mouvement rotatif relatif de l'élément de palier (5) et de la surface d'appui (20) l'un par rapport à l'autre.

4. Auxiliaire de piqûre selon l'une quelconque des revendications 1 à 3, **caractérisé en ce que** l'unité de déclenchement (1) contient au moins un élément d'entraînement en rotation (3) qui est poussé, via un mouvement linéaire de la touche de déclenchement (2), contre ledit au moins un élément de palier (5), et imprime ainsi un mouvement rotatif de l'élément de palier par rapport à la surface d'appui (20), la touche de déclenchement (2) présentant au moins un crochet (11, 19) qui est disposé de telle sorte qu'il vient s'accrocher dans le logement (9) après le mouvement linéaire de la touche de déclenchement (2), si bien que la touche de déclenchement (2) ne peut effectuer un mouvement linéaire en retour et l'élément d'entraînement rotatif (3) reste disposé de telle sorte qu'il bloque, à titre d'élément de blocage, un mouvement rotatif de l'élément de palier (5) par rapport à la surface d'appui (20) en retour dans la première position.

5. Auxiliaire de piqûre selon la revendication 4, **caractérisé en ce que** le crochet (11, 19), après le mouvement linéaire de la touche de déclenchement (2), vient s'accrocher dans un évidement du logement ou derrière une saillie (14, 21) du logement (9).

6. Auxiliaire de piqûre selon l'une quelconque des revendications 4 ou 5, **caractérisé en ce que** le crochet (11, 19), lors du mouvement linéaire de la touche de déclenchement (2), glisse par-dessus une saillie (14) du logement (9) en forme de rampe et vient s'accrocher, après le mouvement linéaire, derrière la saillie (14) en forme de rampe.

7. Auxiliaire de piqûre selon l'une quelconque des revendications 4 à 6, **caractérisé en ce que** le crochet (11, 19) accroche la touche de déclenchement (2) avec le logement (9), avant le déclenchement du mouvement de piqûre de la lancette, afin d'éviter le décrochage de la touche de déclenchement (2) du logement (9).

8. Auxiliaire de piqûre selon l'une quelconque des revendications 4 à 7, **caractérisé en ce que** la touche de déclenchement (2) présente au moins deux crochets (18, 19), au moins un crochet (18) servant à accrocher la touche de déclenchement (2) dans le logement (9) avant le déclenchement du mouvement de piqûre et au moins un crochet supplémentaire (19) servant à accrocher la touche de déclenchement (2) dans le logement (9) après le mouvement linéaire pour empêcher le mouvement linéaire en retour de la touche de déclenchement (2).

9. Auxiliaire de piqûre selon l'une quelconque des revendications 1 à 8, **caractérisé en ce que** l'auxiliaire de piqûre présente une protection stérile amovible (7) qui présente des saillies (23) qui sont disposées de telle sorte que la protection stérile (7), après le mouvement de piqûre de la lancette, peut venir s'insérer pour faire office d'élément de blocage dans l'ouverture (22) du logement (9) et vient s'accrocher, avec ses saillies (23), dans l'ouverture (22) en bloquant l'ouverture (22) du logement (9), si bien qu'un mouvement rotatif de l'élément de palier (5) par rapport à la surface d'appui (20) est empêché par la protection stérile (7).

10. Auxiliaire de piqûre selon l'une quelconque des revendications 1 à 9, **caractérisé en ce que** l'auxiliaire de piqûre comprend, à titre d'élément de blocage, un élément d'aiguillage (27) qui est réalisé pour pouvoir se déplacer dans une direction afin de laisser passer le dispositif de support de lancettes (6) lors d'un mouvement rotatif relatif de l'élément de palier (5) et de la surface d'appui (20) l'un par rapport à l'autre, tout en étant réalisé de façon à pouvoir se déplacer dans une autre direction, seulement jusqu'à une position de blocage, l'élément d'aiguillage (27) bloquant, dans la position de blocage, le dispositif de support de lancettes (6) de telle sorte qu'il empêche un mouvement rotatif en retour dans la première position.

11. Auxiliaire de piqûre selon la revendication 10, **caractérisé en ce que** l'élément d'escamotage (27) est disposé sur le bord de la surface d'appui (20) ou contre le dispositif de support de lancettes (6).
